# EUROPÄISCHE PATENTSCHRIFT

(11) **EP 1 820 663 B1**
(45) Veröffentlichungstag und Bekanntmachung des Hinweises auf die Patenterteilung: **06.04.2011**
(21) Anmeldenummer: 07000867.7
(22) Anmeldetag: 17.01.2007
(51) Int. Cl.: B44C 5/00, A47B 95/00, A61L 2/238

(54) **Profilleiste mit oligodynamisch wirkendem Material**
Strip with oligodynamic material
Baguette avec materiél oligodynamique

(30) Priorität: 20.02.2006 DE 102006008143
(43) Veröffentlichungstag der Anmeldung: 22.08.2007
(73) Patentinhaber: REHAU AG + Co, 95111 Rehau (DE)
(72) Erfinder: Krämer, Uwe, 95111 Rehau (DE); Puchta, Gerd, 95111 Rehau (DE)

(56) Entgegenhaltungen:
- WO-A-01/43788
- WO-A-95/20878
- DE-C1- 19 818 553
- FR-A- 2 863 171
- GB-A- 536 801
- US-A- 4 677 143

## Beschreibung

Die Erfindung betrifft eine Kantenleiste für die Möbelindustrie aus einem polymeren Werkstoff, mit einem Leistenkern und wenigstens einem Dekorelement.

Derartige Kantenleisten sind in einer Vielzahl und in verschiedenen Ausführungsbeispielen aus dem Stand der Technik bekannt.
Diese Kantenleisten haben sich in der Praxis gut bewährt. Gleichwohl besteht ständig ein Bedarf, bestimmte Eigenschaften einer derartigen Kantenleiste bspw. bezüglich der Verarbeitung sowie der Ablagerung aggressiver Stoffe weiter zu verbessern.

Die Erfindung hat sich die Aufgabe gestellt, die Nachteile des Standes der Technik zu überwinden und eine Kantenleiste aufzuzeigen, die kostengünstig und wirtschaftlich herstellbar ist, die eine Ablagerung von aggressiven Stoffen beziehungsweise Mikroorganismen auf ihrer Oberfläche deutlich verringert beziehungsweise gar nicht erst zulässt und bei der die Materialeigenschaften und Dekoreigenschaften erhalten bleiben.

Erfindungsgemäß wird diese Aufgabe durch die kennzeichnenden Merkmale des Anspruches 1 gelöst.
Weitere die Erfindung detailliert beschreibende und ergänzende Merkmale sind in den Unteransprüchen ausgeführt.

WO-A-95/20878 offenbart ein Verfahren zur Herstellung von bakteriziden/fungiziden Kunstoffkörpern.

Die erfindungsgemäße Kantenleiste zeichnet sich dadurch aus, dass der Werkstoff der Profilleiste und/oder des Dekorelementes wenigstens ein oligodynamisch wirkendes Material in einer Menge von 0,05 bis zu 20 % bezogen auf die Gesamtmasse, eingelagert enthält.

Die erfindungsgemäße Kantenleiste zeichnet sich weiterhin dadurch aus, dass der Werkstoff der Profilleiste und/oder des Dekorelementes über den gesamten Querschnitt in räumlich gleich bleibender Verteilung wenigstens ein oligodynamisch wirkendes Material in einer Menge von 0,05 bis zu 20 % bezogen auf die Gesamtmasse, einlagert enthält.

In einer weiteren vorteilhaften Ausführungsform ist die erfindungsgemäße Kantenleiste so ausgeführt, dass der Werkstoff der Kantenleiste und/oder des Dekorelementes wenigstens ein oligodynamisch wirkendes Material in einer Menge von 0,05 bis 20 % bezogen auf die Gesamtmasse, eingelagert enthält, welches vorzugsweise in dem vom Dekorelement abgewandten Bereich konzentriert ist.
Somit kann vorteilhafter Weise erreicht werden, dass die dem Nutzer zugewandte Oberfläche des Werkstoffes der Kantenleiste und/oder des Dekorelementes so ausgebildet ist, dass keine chemisch beziehungsweise biologisch bedingten Verkrustungen beziehungsweise Bewuchse sich anlagern und ausbilden können.

Das oligodynamisch wirkende Material der erfindungsgemäßen Kantenleiste ist in einer weiteren Ausführungsform eine bakterizid und / oder mikrobizid wirkende Metallkomponente. Es liegt jedoch auch im Rahmen der Erfindung, dass das oligodynamisch wirkende Material der erfindungsgemäßen Kantenleiste und/oder des Dekorelementes eine metallorganische Verbindung, wie bspw. Zinkpyrithion und / oder eine metallanorganische Verbindung, wie bspw. ein Silberzeolith.

Die erfindungsgemäße Kantenleiste weist eine signifikante bakterizide Wirkung gegenüber Pseudomonas aeruginosa, Salmonella typhimurium, Salmonella choleraesius, Listeria monocytogenes, Staphylococcus aureus, Alcaligenes faecalis, Escherichia coli und Enterococcus faecalis auf. Es konnte weiterhin vorteilhaft festgestellt werden, dass die erfindungsgemäße Profilleiste eine signifikante fungizide Wirkung gegenüber Candida albicans aufweist.

Bei der erfindungsgemäßen Kantenleiste ist die Metallkomponente eine Metallionen abgebende Substanz, die durch den Kontakt mit bspw. einer Flüssigkeit, insbesondere beim dem Abwaschen der Profilleiste, die Metallionen freisetzt.
Es konnte überraschend gefunden werden, dass die Metallionen abgebende Substanz ein Salz, Oxid, Carbid, eine metallorganische und / oder metallanorganische Verbindung ist, die aus der Gruppe Silber, Gold, Kupfer, Zink, Blei, Arsen, Cadmium ausgewählt ist.

Entgegen der Lehre des Standes der Technik, derartige Kantenleisten mit zusätzlichen aufwendigen Schutzschichten zu versehen, konnte überraschend gefunden werden, dass die metallabgebende Substanz aus der Gruppe der Zeolithe ausgewählt ist, wobei diese sowohl natürlich vorkommende als auch industriell hergestellte Zeolithe sind.

Die Kantenleiste zeichnet sich außerdem erfindungsgemäß dadurch aus, dass ein Teil der im Zeolith enthaltenen Metalle durch wenigsten eine Art von Ionen austauschbaren Metallen ersetzt ist, die aus der Gruppe Silber, Gold, Kupfer, Zink, Blei, Arsen, Cadmium ausgewählt sind. Hier haben sich beispielsweise Wirkstoffe auf der Basis von Sodium Alumosilicaten ausgezeichnet.
Ein weiterer Vorteil der erfindungsgemäßen Kantenleiste wird dadurch gesehen, dass eine langfristige Sicherstellung des Nichtbildens bzw. Anwachsens von Belägen in bspw. Küchenanwendungen sichergestellt ist, da die Metallionen abgebende Substanz in einer mittleren Korngröße von etwa 6 µm in dem Werkstoff der Profilleiste und/oder des Dekorelementes so eingearbeitet werden kann, dass sowohl eine Verteilung über den gesamten Querschnitt in räumlich gleichmäßiger Verteilung, als auch eine Verteilung vorzugsweise in dem vom Dekorelement abgewandten Bereiches der Kantenleiste realisierbar ist.
Das Dekorelement ist dabei in an sich bekannter Weise bspw. als Lackbeschichtung ausgebildet. Diese sind an sich bekannterweise UV- härtende Lacke, wie Polyacrylate; lösungsmittelhaltige Lacke, wie PUR- bzw. Acrylat- basierende Lacke bzw. wässrige Lacke, wie PUR- basierende Lacke.

Es hat sich als vorteilhaft herausgestellt, dass bei der erfindungsgemäßen Kantenleiste der Werkstoff der Kantenleiste ein Polyolefin wie bspw. PP (Polypropylen), ein ABS (AcrylnitrilButadien-Styrol), ein PMMA (Polymethylmethacrylat), ein PVC (Polyvinylchlorid), ein PET (Polyethylentherephthalat) ein Styrolcopolymeres und dergleichen sein kann. Durch den Einsatz der erfindungsgemäßen Kantenleiste können die Reinigungsaufwendungen beim Einsatz bspw. in Küchen oder in medizinischen Bereichen wie Arztpraxen reduziert werden.

Bei der Verwendung eines Werkstoffes der Kantenleiste und/oder des Dekorelementes mit wenigsten ein oligodynamisch wirkendem Material in einer Menge von 0,05 bis 20 % bezogen auf die Gesamtmasse in einer Kantenleiste zum Anbringen an eine Möbelbauplatte, mit mindestens einem Dekorelement und einer Kantenleiste aus einem polymeren Werkstoff, wobei das Dekorelement mit der Kantenleiste verbunden ist, kann eine signifikante Verbesserung der Eigenschaften bezüglich der Verarbeitung sowie der Ablagerung aggressiver Stoffe durch die Kantenleiste realisiert werden.

Weiterhin vorteilhaft wird gesehen, dass auch bereits bei Kantenleisten, welche mit den Nachteilen des bekannten Standes zur Technik behaftet sind, eine kostengünstige "Sanierung" realisiert werden kann, die zu den gleichen Vorteilen wie eine ursprünglich montierte erfindungsgemäße Kantenleiste führt, indem ein erfindungsgemäßes Dekorelement auf die Kantenleiste aufgebracht wird.

Es liegt jedoch auch im Rahmen der Erfindung, dass der Werkstoff der Kantenleiste in einer Menge von 0,05 bis zu 20 % bezogen auf die Gesamtmasse Fungizide wie beispielsweise DCOIT (4,5-Dichlor-2-N-octyl-4-isothiazolin-3-on Dichloroctylisothiazolinon), OIT (2-N-octyl-4-isothiazolin-3-on), Bethoxazin (3-Benzo[b]thien-2-yl-5,6-dihydro-1,4,2-oxathiazin,4-oxid), Fluorfolpet (2-(Dichlorfluormethylthio)-isoindol-1,3-dion N-Fluordichlormethylthiophthalimid Fluorcaptan Fluorphaltan), OBPA (10,10'-Oxybispenoxyarsin) beziehungsweise BCM (Methyl-N-(2-benzimidazolyl) -carbamat Benzimidazolylcarbaminsäuremethylester Carbendazim) aufweist.

Im Folgenden soll an den die Erfindung nicht einschränkenden Beispielen, diese näher beschrieben werden.

### Beispiel 1:

Die erfindungsgemäße Kantenleiste weist einen Leistenkern auf, bestehend aus:

| | | |
|---|---|---|
| 98 bis 99 % | Polypropylen | |
| 0,5 bis 2,0 % | Silbersalz X₂ₙO- Na₂O- Al₂O₃- 2SiO₂ | X= Ag⁺ |

### Beispiel 2:

Die erfindungsgemäße Kantenleiste weist eine Leistenkern auf, bestehend aus:

| | | |
|---|---|---|
| 94 - 99,4 % | Polyvinylchlorid | |
| 0,1 bis 1,0% | Silbersalz X₂ₙO- Na₂O- Al₂O₃- 2SiO₂ | X= Ag⁺ |
| 0,5 bis 5 % | OBPA (10,10'-Oxybispenoxyarsin) | |

### Beispiel 3:

Die erfindungsgemäße Kantenleiste weist eine Leistenkern auf, bestehend aus:

| | | |
|---|---|---|
| 95 bis 99,9 % | Styrolcopolymer | |
| 0,1 bis 5% | Silbersalz X₂ₙO- Na₂O- Al₂O₃- 2SiO₂ | X= Ag⁺ |

und ein Dekorelement, bestehend aus:

| | | |
|---|---|---|
| 93 bis 99 % | Polyacrylat | |
| 5,5 % | Silicate | |
| 0,5 bis 1,0 % | Zinkpyrithion | |

## Patentansprüche

1. Kantenleiste für die Möbelindustrie aus einem polymeren Werkstoff, mit einem Leistenkern und wenigstens einem Dekorelement, **dadurch gekennzeichnet, dass** der Werkstoff der Kentenleiste und/oder des Dekorelementes wenigstens ein oligodynamisch wirkendes Material in einer Menge von 0,05 bis zu 20 %, bezogen auf die Gesamtmasse, eingelagert enthält.

2. Kentenleiste nach Anspruch 1, **dadurch gekennzeichnet, dass** der Werkstoff der Kentenleiste und/oder des Dekorelementes über seinen gesamten Querschnitt in räumlich gleichmäßiger Verteilung wenigstens ein oligodynamisch wirkendes Material in einer Menge von 0,05 bis zu 20 %, bezogen auf die Gesamtmasse, eingelagert enthält.

3. Kentenleiste nach Anspruch 1, **dadurch gekennzeichnet, dass** der Werkstoff der Kentenleiste und/oder des Dekorelementes wenigstens ein oligodynamisch wirkendes Material in einer Menge von 0,05 bis zu 20 %, bezogen auf die Gesamtmasse, eingelagert enthält, welches vorzugsweise in dem vom Dekorelement abgewandten Bereich konzentriert ist.

4. Kentenleiste nach einem der Ansprüche 1 bis 3; **dadurch gekennzeichnet, dass** das oligodynamisch wirkende Material eine bakterizid und / oder microbizid wirkende Metallkomponente ist.

5. Kentenleiste nach einem der Ansprüche 1 bis 3, **dadurch gekennzeichnet, dass** das oligodynamisch wirkende Material eine metallorganische und / oder metallanorganische Verbindung ist.

6. Kentenleiste nach einem der Ansprüche 1 bis 5, **dadurch gekennzeichnet, dass** das oligodynamisch wirkende Material eine signifikante Wirkung gegenüber Pseudomonas aeruginosa, Salmonella typhimurium, Salmonella choleraesius, Listeria monocytogenes, Staphylococcus aureus, Alcaligenes faecalis, Escherichia coli, Candida albicans, Enterococcus faecalis aufweist.

7. Kentenleiste nach einem der Ansprüche 1 bis 6, **dadurch gekennzeichnet, dass** die Metallkomponente eine metallorganische oder Metallionen abgebende Substanz ist.

8. Kentenleiste nach einem der Ansprüche 1 bis 7, **dadurch gekennzeichnet, dass** die Metallkomponente durch den Kontakt mit der Flüssigkeit, insbesondere Wasser, die Metallionen freisetzt.

9. Kentenleiste nach einem der Ansprüche 1 bis 8, **dadurch gekennzeichnet, dass** die Metallionen abgebende Substanz ein Salz, Oxid, Carbid, eine metallorganische und / oder metallanorganische Verbindung ist, die aus der Gruppe Silber, Gold, Kupfer, Zink, Blei, Arsen, Cadmium ausgewählt ist.

10. Kentenleiste nach einem der Ansprüche 1 bis 9, **dadurch gekennzeichnet, dass** die Metallionen abgebende Substanz aus der Gruppe der Zeolithe und / oder der ChelatKomplexe ausgewählt ist.

11. Kentenleiste nach einem der Ansprüche 1-10, **dadurch gekennzeichnet, dass** ein Teil der im Zeolith und/ oder dem Chelat-Komplex enthaltenen Metalle durch wenigstens eine Art von Ionen austauschbaren Metallen ersetzt ist, die aus der Gruppe Silber, Gold, Kupfer, Zink, Blei, Arsen, Cadmium ausgewählt sind.

12. Kentenleiste nach einem der Ansprüche 7 bis 11, **dadurch gekennzeichnet, dass** die Metallionen abgebende Substanz eine mittlere Korngröße von etwa 0,1 bis 10 µm aufweist.

13. Kentenleiste nach einem der Ansprüche 1 bis 11, **dadurch gekennzeichnet, dass** der Werkstoff der Kentenleiste ein Polyolefin ist.

14. Kentenleiste nach einem der Ansprüche 1 bis 11, **dadurch gekennzeichnet, dass** der Werkstoff der Kentenleiste ein Styrolcopolymer ist.

15. Kentenleiste nach einem der Ansprüche 1 bis 11, **dadurch gekennzeichnet, dass** der Werkstoff der Klentenleiste ein Polymethylmethacrylat ist.

16. Kentenleiste nach einem der Ansprüche 1 bis 11, **dadurch gekennzeichnet, dass** der Werkstoff der ein Polyethylentherephthalat ist.

17. Kentenleiste nach einem der Ansprüche 1 bis 11, **dadurch gekennzeichnet, dass** der Werkstoff der Kentenleiste ein Polyvinylchlorid ist.

## Claims

1. Edge strip for the furniture industry made of a polymeric material, with a strip core and at least one decorative element, **characterized in that** the material of the edge strip and/or of the decorative element comprises at least one oligodynamic material in an amount of from 0.05 up to 20%, based on the total mass, in incorporated form.

2. Edge strip according to Claim 1, **characterized in that** the material of the edge strip and/or of the decorative element comprises, in incorporated from, over its entire cross section in spatially uniform distribution, at least one oligodynamic material in an amount of from 0.05 up to 20%, based on the total mass.

3. Edge strip according to Claim 1, **characterized in that** the material of the edge strip and/or of the decorative element comprises, in incorporated form, at least one oligodynamic material in an amount of from 0.05 up to 20%, based on the total mass, which is concentrated preferably in the area facing away from the decorative element.

4. Edge strip according to one of Claims 1 to 3, **characterized in that** the oligodynamic material is a bactericidal and/or microbicidal metal component.

5. Edge strip according to one of Claims 1 to 3, **characterized in that** the oligodynamic material is an organometallic and/or inorganometallic compound.

6. Edge strip according to one of Claims 1 to 5, **characterized in that** the oligodynamic material has a significant effect towards Pseudomonas aeruginosa, Salmonella typhimurium, Salmonella choleraesius, Listeria monocytogenes, Staphylococcus aureus, Alcaligenes faecalis, Escherichia coli, Candida albicans, Enterococcus faecalis.

7. Edge strip according to one of Claims 1 to 6, **characterized in that** the metal component is an organometallic substance or a substance releasing metal ions.

8. Edge strip according to one of Claims 1 to 7, **characterized in that** the metal component releases the metal ions as a result of contact with the liquid, in particular water.

9. Edge strip according to one of Claims 1 to 8, **characterized in that** the substance releasing metal ions is a salt, oxide, carbide, an organometallic and/or inorganometallic compound which is selected from the group silver, gold, copper, zinc, lead, arsenic, cadmium.

10. Edge strip according to one of Claims 1 to 9, **characterized in that** the substance releasing metal ions is selected from the group of zeolites and/or chelate complexes.

11. Edge strip according to one of Claims 1-10, **characterized in that** some of the metals present in the zeolite and/or the chelate complex are replaced by at least one type of ion exchangeable metals which are selected from the group silver, gold, copper, zinc, lead, arsenic, cadmium.

12. Edge strip according to one of Claims 7 to 11, **characterized in that** the substance releasing metal ions has an average particle size of from about 0.1 to 1 µm.

13. Edge strip according to one of Claims 1 to 11, **characterized in that** the material of the edge strip is a polyolefin.

14. Edge strip according to one of Claims 1 to 11, **characterized in that** the material of the edge strip is a styrene copolymer.

15. Edge strip according to one of Claims 1 to 11, **characterized in that** the material of the edge strip is a polymethyl methacrylate.

16. Edge strip according to one of Claims 1 to 11, **characterized in that** the material of the edge strip is a polyethylene terephthalate.

17. Edge strip according to one of Claims 1 to 11, **characterized in that** the material of the edge strip is a polyvinyl chloride.

## Revendications

1. Baguette de lisière pour l'industrie du meuble, en un matériau polymère, avec une âme de baguette et au moins un élément décoratif, **caractérisée en ce que** le matériau de la baguette de lisière et/ou de l'élément décoratif contient, emmagasiné dans ce dernier, au moins une matière à effet oligodynamique dans une quantité de 0,05 à 20 % par rapport à la masse totale.

2. Baguette de lisière selon la revendication 1, **caractérisée en ce que** le matériau de la baguette de lisière et/ou de l'élément décoratif contient, emmagasiné dans ce dernier, sur l'ensemble de sa section transversale, en répartition dans l'espace régulière, au moins une matière à effet oligodynamique dans une quantité de 0,05 à 20 % par rapport à la masse totale.

3. Baguette de lisière selon la revendication 1, **caractérisée en ce que** le matériau de la baguette de lisière et/ou de l'élément décoratif contient, emmagasiné dans ce dernier, au moins une matière à effet oligodynamique, dans une quantité de 0,05 à 20 % par rapport à la masse totale, laquelle est concentrée de préférence dans la zone opposée à l'élément décoratif.

4. Baguette de lisière selon l'une quelconque des revendications 1 à 3, **caractérisée en ce que** la matière à effet oligodynamique est un composant métallique à effet bactéricide et/ou microbicide.

5. Baguette de lisière selon l'une quelconque des revendications 1 à 3, **caractérisée en ce que** la matière à effet oligodynamique est un composé métallo-organique et/ou métallo-anorganique.

6. Baguette de lisière selon l'une quelconque des revendications 1 à 5, **caractérisée en ce que** la matière à effet oligodynamique fait preuve d'une action significative contre le pseudomonas aeruginosa, la salmonella typhimurium, la salmonella choleraesius, la listeria monocytogenes, le staphylococcus aureus, l'alcaligenes faecalis, l'escherichia coli, le candida albicans, l'enterococcus faecalis.

7. Baguette de lisière selon l'une quelconque des revendications 1 à 6, **caractérisée en ce que** le composant métallique est une substance métallo-organique ou restituant des ions métalliques.

8. Baguette de lisière selon l'une quelconque des revendications 1 à 7, **caractérisée en ce que** le composant métallique libère les ions métalliques par contact avec le liquide, notamment de l'eau.

9. Baguette de lisière selon l'une quelconque des revendications 1 à 8, **caractérisée en ce que** la substance restituant des ions métalliques est un sel, un oxyde, un carbure, un composé métallo-organique et/ou métallo-anorganique, choisi dans le groupe comportant l'argent, l'or, le cuivre, le zinc, le plomb, l'arsenic, le cadmium.

10. Baguette de lisière selon l'une quelconque des revendications 1 à 9, **caractérisée en ce que** la substance restituant des ions métalliques est choisie dans le groupe des zéolithes et/ou des complexes chélates.

11. Baguette de lisière selon l'une quelconque des revendications 1 à 10, **caractérisée en ce qu'**une partie des métaux contenus dans la zéolithe et/ou dans le complexe chélate est remplacée par au moins un type de métal échangeur d'ions, qui est choisi dans le groupe comportant l'argent, l'or, le cuivre, le zinc, le plomb, l'arsenic, le cadmium.

12. Baguette de lisière selon l'une quelconque des revendications 7 à 11, **caractérisée en ce que** la substance restituant des ions métalliques présente une grosseur moyenne de grains d'environ 0,1 à 1 µm.

13. Baguette de lisière selon l'une quelconque des revendications 1 à 11, **caractérisée en ce que** le matériau de la baguette de lisière est une polyoléfine.

14. Baguette de lisière selon l'une quelconque des revendications 1 à 11, **caractérisée en ce que** le matériau de la baguette de lisière est un copolymère de styrène.

15. Baguette de lisière selon l'une quelconque des revendications 1 à 11, **caractérisée en ce que** le matériau de la baguette de lisière est un polyméthyl-méthacrylate.

16. Baguette de lisière selon l'une quelconque des revendications 1 à 11, **caractérisée en ce que** le matériau de la baguette de lisière est un polyéthylène-téréphtalate.

17. Baguette de lisière selon l'une quelconque des revendications 1 à 11, **caractérisée en ce que** le matériau de la baguette de lisière est un chlorure de polyvinyle.
